# EUROPÄISCHE PATENTSCHRIFT

(11) **EP 0 942 965 B1**
(45) Veröffentlichungstag und Bekanntmachung des Hinweises auf die Patenterteilung: **31.10.2007**
(21) Anmeldenummer: 97953733.9
(22) Anmeldetag: 03.12.1997
(51) Int. Cl.: C12N 9/14, A01H 1/00, C12N 1/20, C12N 15/55

(54) **NEUES GEN FÜR EINE AMINOSÄURE-DEACETYLASE MIT SPEZIFITÄT FÜR N-ACETYL-L-PHOSPHINOTHRICIN, IHRE ISOLIERUNG UND VERWENDUNG**
NOVEL GENE CODING FOR AN AMINO ACID DEACETYLASE WITH SPECIFICITY FOR N-ACETYL-L-PHOSPHINOTHRICIN, THEIR ISOLATION AND THEIR USE
NOUVEL GENE CODANT POUR UNE DESACETYLASE D'AMINOACIDE A SPECIFICITE POUR N-ACETYLE-L-PHOSPHINOTHRICINE, SON ISOLATION ET SON UTILISATION

(30) Priorität: 16.12.1996 DE 19652284
(43) Veröffentlichungstag der Anmeldung: 22.09.1999
(73) Patentinhaber: Bayer CropScience AG, 40789 Monheim (DE)
(72) Erfinder: BARTSCH, Klaus, D-61462 Königstein (DE); KRIETE, Guido, D-33790 Halle (DE); BROER, Inge, D-18184 Roggentin (DE); PÜHLER, Alfred, D-33793 Bielefeld (DE)
(86) Internationale Anmeldenummer: PCT/EP1997/006755
(87) Internationale Veröffentlichungsnummer: WO 1998/027201

(56) Entgegenhaltungen:
- DE-A- 4 126 414
- DE-A- 4 308 061
- DE-A- 19 639 463
- FR-A- 2 258 448
- KRIETE G ET AL: "MALE STERILITY IN TRANSGENIC TOBACCO INDUCED BY TAPETUM-SPECIFIC DEACETYLATION OF THE EXTERNALLY APPLIED NON-TOXIC COMPOUND N-ACETYL-L-PHOSPHINOTHRICIN" PLANT JOURNAL, Bd. 9, Nr. 6, 1996, Seiten 809-818, XP002053694

### SEQUENZPROTOKOLL

(1) ALGEMEINE INFORMATION:
   (i) ANMELDER:
      (A) NAME: Hoechst Schering AgrEvo GmbH
      (B) STRASSE: -
      (C) ORT: Frankfurt
      (D) BUNDESLAND: -
      (E) LAND: Deutschland
      (F) POSTLEITZAHL: 65926
      (G) TELEPHON: 069-305-5596
      (H) TELEFAX: (+69) 357175
      (I) TELEX: -
   (ii) ANMELDETITEL: Neue Gene codierend fuer Aminosaeure-Deacetylen mit Spezifitaet fuer N-Acetyl-L-Phosphinothricin, ihre Isolierung und Verwendung
   (iii) ANZAHL DER SEQUENZEN: 4
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.25 (EPA)
(2) INFORMATION ZU SEQ ID NO: 1:
   (i) SEQUENZ CHARAKTERISTIKA:
      (A) LÄNGE: 1365 Basenpaare
      (B) ART: Nukleinsäure
      (C) STRANGFORM: Einzel
      (D) TOPOLOGIE: linear
   (ii) ART DES MOLEKÜLS: DNS (genomisch)
   (ix) MERKMALE:
      (A) NAME/SCHLÜSSEL: exon
      (B) LAGE: 1..1365
   (xi) SEQVENZBESCHREIBUNG: SEQ ID NO: 1: 
ATGATCCGCA AGACCGTTCT GTTGACTGCG CTGTCCTGCG CCCTGGCCTC CGCGACAGCC 60

ACCGCCGCCG AAGGCCAGCG GCCCGAGGTG GAGGCCGCCG CCGCGCGCCT GCAGCGGCAG 120

GTGGTGGAGT GGCGCCGCGA TTTCCACCAG CATCCGGAGC TGTCCAACCG CGAGGTGCGC 180

ACCGCCGCCA AGGTGGCCGA GCGCCTGCGC GCGATGGGCC TGCAACCGCA GACCGGCGTC 240

GCCGTGCACG GCGTGGTGGC GATCATCAAG GGCGCCCTGC CGGGGCCGAA GATCGCCCTG 300

CGCGCGGACA TGGACGCGCT GCCGGTGACC GAACAGACCG GGCTGCCGTT CGCCTCCACC 360

GCCACGGCCG AGTACCGCGG CGAGAAGGTC GGGGTGATGC ATGCCTGCGG CCACGACGCC 420

CATACCGCCA CCCTGCTCGG CGTGGCCGAC GCGCTGGTGG CCATGCGCGA CACGCTGCCC 480

GGCGAAGTAA TGCTGATCTT CCAGCCGGCC GAGGAAGGCG CGCCGCCGCC GGAGCAGGGC 540

GGTGCCGAGC TGATGCTCAA GGAGGGGCTG TTCAAGGAGT TCAAGCCGGA GGCGGTGTTC 600

GGCCTGCACG TGTTCTCCAG CGTCCAGGCC GGGCAGATCG CCGTGCGCGG CGGCCCGCTG 660

ATGGCCGCCT CCGACCGCTT CGCCATCACC GTCAACGGCC GCCAGACCCA TGGCTCGGCG 720

CCCTGGAACG GCATCGATCC GATTGTCGCC GCCTCCGACC TGATCGGCAC CGCGCAGACC 780

ATCGTCAGCC GCCGCGCCAA CCTGTCCAAG CAGCCGGCGG TGCTGACCTT CGGCGCGATC 840

AAGGGCGGCA TCCGCTACAA CATCATCCCC GACTCGGTGG AGATGGTCGG CACCATCCGC 900

ACCTTCGACC CGGACATGCG CAAGCAGATC TTCGCCGACT TGCGCAACGT CGCCGAGCAC 960

ACCGCTGCCG CATGGCGCCA CCGCCACCAC CGACATCTAC GAGAAGGACG GCAACCCGGC 1020

CACGGTCAAC GACCCGGCGC TGACCGCGCG CATGCTGCCC AGCCTGCAGG CCGTGGTCGG 1080

CAAGGACAAC GTCTACGAGC CGCCGCTGCA GATGGGCTCG GAGGACTTCT CGCTGTATGC 1140

GCAGCAGGTG CCGGCGATGT TCTTCTTCGT CGGCTCCACC GGCGCGGGCA TCGACCCGGC 1200

CACCGCGCCC AGCAACCACT CGCCGAAGTT CCTGCTCGAC GAGAAGGCGC TGGACGTGGG 1260
 
CCTGCGCGCG CTGCTGCAGG TGTCGCTGGA CTATCTGCAC GGTGGCAAGG CGGGGTGACC 1320

CCTGCCAGTA TCGTGCCCCC GATACGGAAG AAGGACCTCC CATGA 1365
(2) INFORMATION ZU SEQ ID NO: 2:
   (i) SEQUENZ CHARAKTERISTIKA:
      (A) LÄNGE. 454 Aminosäuren
      (B) ART: Aminosäure
      (C) STRANGFORM: Einzel
      (D) TOPOLOGIE: linear
   (ii) ART DES MOLEKÜLS: Protein
   (ix) MERKMALE;
      (A) NAME/SCHLÜSSEL: Protein
      (B) LAGE: 1..454
   (xi) SEQUENZBESCHREIBUNG: SEQ ID NO: 2:

Met Ile Arg Lys Thr Val Leu Leu Thr Ala Leu Ser Cys Ala Leu Ala
1               5                   10                  15

Ser Ala Thr Ala Thr Ala Ala Glu Gly Gln Arg Pro Glu Val Glu Ala
            20                  25                  30

Ala Ala Ala Arg Leu Gln Arg Gln Val Val Glu Trp Arg Arg Asp Phe
        35                  40                  45

His Gln His Pro Glu Leu Ser Asn Arg Glu Val Arg Thr Ala Ala Lys
    50                  55                  60

Val Ala Glu Arg Leu Arg Ala Met Gly Leu Gln Pro Gln Thr Gly Val
65                  70                  75                  80

Ala Val His Gly Val Val Ala Ile Ile Lys Gly Ala Leu Pro Gly Pro
                85                  90                  95

Lys Ile Ala Leu Arg Ala Asp Met Asp Ala Leu Pro Val Thr Glu Gln
            100                 105                 110

Thr Gly Leu Pro Phe Ala Ser Thr Ala Thr Ala Glu Tyr Arg Gly Glu
        115                 120                 125

Lys Val Gly Val Met His Ala Cys Gly His Asp Ala His Thr Ala Thr
    130                 135                 140

Leu Leu Gly Val Ala Asp Ala Leu Val Ala Met Arg Asp Thr Leu Pro
145                 150                 155                 160

Gly Glu Val Met Leu Ile Phe Gln Pro Ala Glu Glu Gly Ala Pro Pro
                165                 170                 175

Pro Glu Gln Gly Gly Ala Glu Leu Met Leu Lys Glu Gly Leu Phe Lys
            180                 185                 190

Glu Phe Lys Pro Glu Ala Val Phe Gly Leu His Val Phe Ser Ser Val
        195                 200                 205

Gln Ala Gly Gln Ile Ala Val Arg Gly Gly Pro Leu Met Ala Ala Ser
    210                 215                 220

Asp Arg Phe Ala Ile Thr Val Asn Gly Arg Gln Thr His Gly Ser Ala
225                 230                 235                 240

Pro Trp Asn Gly Ile Asp Pro Ile Val Ala Ala Ser Asp Leu Ile Gly
                245                 250                 255

Thr Ala Gln Thr Ile Val Ser Arg Arg Ala Asn Leu Ser Lys Gln Pro
            260                 265                 270

Ala Val Leu Thr Phe Gly Ala Ile Lys Gly Gly Ile Arg Tyr Asn Ile
        275                 280                 285

Ile Pro Asp Ser Val Glu Met Val Gly Thr Ile Arg Thr Phe Asp Pro
    290                 295                 300

Asp Met Arg Lys Gln Ile Phe Ala Asp Leu Arg Asn Val Ala Glu His
305                 310                 315                 320

Thr Ala Ala Ala Trp Arg His Arg His His Arg His Leu Arg Glu Gly
                325                 330                 335

Arg Gln Pro Gly His Gly Gln Arg Pro Gly Ala Asp Arg Ala His Ala
            340                 345                 350

Ala Gln Pro Ala Gly Arg Gly Arg Gln Gly Gln Arg Leu Arg Ala Ala
        355                 360                 365

Ala Ala Asp Gly Leu Gly Gly Leu Leu Ala Val Cys Ala Ala Gly Ala
    370                 375                 380

Gly Asp Val Leu Leu Arg Arg Leu His Arg Arg Gly His Arg Pro Gly
385                 390                 395                 400

His Arg Ala Gln Gln Pro Leu Ala Glu Val Pro Ala Arg Arg Glu Gly
                405                 410                 415

Ala Gly Arg Gly Pro Ala Arg Ala Ala Ala Gly Val Ala Gly Leu Ser
            420                 425                 430

Ala Arg Trp Gln Gly Gly Val Thr Pro Ala Ser Ile Val Pro Pro Ile
        435                 440                 445

Arg Lys Lys Asp Leu Pro
    450

## Beschreibung

Das Konzept einer chemisch induzierbaren, reversiblen männlichen Sterilität bei Pflanzen durch Antheren-spezifische Expression einer N-Acetyl-Phosphinothricin (N-Acetyl-PPT)-spezifischen Deacetylase wird in der Europäischen Patentanmeldung EP 531 716 beschrieben. Die dabei verwendeten Deacetylase-Gene aus Streptomyces viridochromogenes [N-Acetyl-L-Phosphinothricyl-alanylalanin (N-Acetyl-PTT)-Deacetylase, dea] bzw. argE aus Escherichia coli (N-Acetyl-L-Ornithin-Deacetylase) codieren Proteine mit Spezifität für N-Acetyl-L-PPT. Für beide Gene konnten bei Tapetum-spezifischer Expression in Pflanzen, das Auftreten männlich steriler Blüten nach Behandlung von Einzelknospen mit N-Acetyl-L-PPT gezeigt werden. Für eine erfolgreiche Anwendung dieses Systems insbesondere bei der Behandlung von ganzen Pflanzen mit N-Acetyl-PPT unter praxisrelevanten Bedingungen ist es von Vorteil, Deacetylasen mit hoher Substataffinität einsetzen zu können. Daher wurde nach weiteren Deacetylasen mit hoher Affinität für N-Acetyl-PPT gesucht.

Der hier beschriebenen Anmeldung liegt somit die Aufgabe zugrunde, DNA-Moleküle zur Verfügung zu stellen, die für Deacetylasen kodieren. Mit diesen Deacetylasen ist es möglich, Pflanzen mit gezielt zerstörbaren Pflanzenteilen herzustellen. Von besonderem Interesse ist das Herstellen von männlich oder weiblich sterilen Pflanzen. Die Aufgabe wird durch die Bereitstellung der in den Patentansprüchen bezeichneten Ausführungsformen gelöst.

Die Erfindung betrifft DNA-Moleküle, die für Deacetylasen oder Proteine mit der biologischen Aktivität einer Deacetylase kodieren. Die enzymatischen Eigenschaften dieser Proteine werden in den Beispielen beschrieben.

Die Erfindung betrifft außerdem transgene Pflanzenzellen, die mit dem erfindungsgemäßen DNA-Molekül transformiert wurden. Die transgenen Pflanzenzellen können nach bekannten Techniken hergestellt werden und zu ganzen Pflanzen regeneriert werden.

Die Erfindung betrifft DNA-Moleküle codierend ein Protein mit der biologischen Aktivität einer N-Acetyl-PPT-Deacetylase.

Die Erfindung betrifft insbesondere DNA-Moleküle codierend ein Protein mit der biologischen Aktivität einer N-Acetyl-PPT-Deacetylase ausgewählt aus der Gruppe bestehend aus
a) DNA-Molekülen, die für ein Protein mit der unter SEQ ID No 2 angegebenen Aminosäuresequenz und Fragmenten und/oder Derivaten davon kodieren;
b) DNA-Molekülen, die für eine unter der SEQ ID No 1 angegebenen Nukleotidsequenz kodieren.

Die Erfindung betrifft außerdem den nach dem in dieser Anmeldung beschriebenen Verfahren identifizierten und hinterlegten Mikroorganismus Stenotrophomonas sp. (DSM 9734)

Gegenstand der Erfindung sind insbesondere Pflanzenzellen oder Pflanzen, die die erfindungsgemäßen DNA-Moleküle enthalten.

Von besonderen Interessen sind Verfahren zur Herstellung von Pflanzen mit gezielt zerstörbaren Teilen durch spezifische Expression eines Deacetylasegens sowie Verfahren zur Herstellung männlich oder weiblich steriler Pflanzen durch spezifische Expression eines Deacetylasegens.

Die vorliegende Anmeldung betrifft desweiteren:
- die DNA-Moleküle, die für Enzyme mit hoher N-Acetyl-PPT-Deacetylase-Aktivität kodieren
- die Proteine, die von diesen Genen kodiert werden
- die Expression dieser Deacetylase-Gene in Pflanzen,

Aus Bodenproben lassen sich in Mineralmedium mit Chitin als alleiniger C-Quelle Bakterien anreichern, die in der Lage sind, N-Acetyl-PPT mit hoher Effektivität zu spalten. Auf diese Weise konnten 2 Bakterienstämme als Reinkulturen isoliert werden: Stenotrophomonas sp. (DSM-Hinterlegungs-Nr. DSM 9734) und Comamonas acidovorans (DSM-Hinterlegungs Nr. 11070).

Die Erfindung betrifft somit ferner:
1. Eine Deacetylase mit
   - einem Molekulargewicht von 20.000 bis 100.000 Dalton
   - einem pH Optimum von 6,5 - 10,0
   - einer Substratspezifität gegenüber L-N-Acetyl-Phosphinothricin.
2. Die Verwendung der unter 1. charakterisierten Deacetylase zur Herstellung männlich steriler Pflanzen.

Die Erfindung betrifft insbesondere ein Enzym, das ein Temperaturoptimum hat, das zwischen 30°C und 50°C liegt

Dar für die Deacetylase codierende Gen wurde in E. coli kloniert. Im Falle des deac 1-Gens aus Stenotrophomonas sp. wurde eine Phagemid-Expressionsbank aus genomischer DNA in E. coli auf N-Acotyl-PPT-spezifischer Deacetylase-Aktivität gescreent.

Die aus der DNA-Sequenz abgeleitete Aminosäuresequenz besitzet außerdem Homologie zu Hippurat-Hydrolasen, wie sie aus Proteindatenbanken bekannt sind.

Die hohe Substrataffinität des deac 1-Proteins für N-Acetyl-L-PPT (Kₘ = 670 *µ*M) macht sich in den transgenen Pflanzen durch eine hohe Empfindlichkeit des Gewebes gegen diese Substanz bemerkbar. So können bei konstitutiver Expression des deac-Gens Pflanzen erhalten werden, deren Blätter noch auf Konzentrationen bis zu 0,4 mg/ml N-Acetyl-D,L-PPT (= 0,2 mg/ml L-Enantiomer) sensitiv reagieren. Bei Tapetum-spezifischer Expression wurde die induktion männlich steriler Blüten durch Behandlung der Knospen mit 2 mg/ml N-Acetyl-D,L-PPT (= 1 mg/ml L-Enantiomer) erreicht. Die beschriebenen Ergebnisse beziehen sich auf Gewächshauspflanzen. Auf Grund der niedrigen Substanzkonzentrationen ist bei Bedarf unter Freilandbedingungen eine Höherdosierung um das 5-10fache ohne weiteres möglich.

Die anschließenden Beispiele dienen dazu, die Erfindung weitergehend zu erläutern. Prozentangaben beziehen sich, wenn nicht anders angegeben, auf das Gewicht. Ferner wird die Erfindung in den Patentansprüchen definiert.

### Beispiel 1: Isolierung und Identifizierung von Bodenmikroorganismen mit einer N-Acetyl-PPT-spezifischen Deacetylase-Aktivität

Je 1 g Boden (sandiger Lehm, Schwanheimer Düne) wurde mit 10 mM NaCl, 10 mM NaPhosphat-Puffer, pH = 7,0 für 1 h bei Raumtemperatur extrahiert. Zur Selektion verschiedener Gruppen von Mikroorganismen wurden die Bodenüberstände in folgende Flüssigmedien überimpft:
(1) MS1-Medium (für Eubakterien):
   5 mM Glucose
   5 mM Succinat
   10 mM Glycerin
   1 g/l NH₄Cl
   50 ml/l Lösung A
   25 ml/l Lösung B
   Lösung A: 50 g/l K₂HPO₄
   Lösung B: 2,5 g/l MgSO₄
   0,5 g/l NaCl
   25 ml/l Spurenelemente
(2) Chitin-Medium (für Actino- und Streptomyceten, sowie chitinovore Bakterien):
   10 g/l Krabbenchitin
   1 g/l (NH₄)₂SO₄
   0,5 g/l MgSO₄
   50 ml/l Lösung A
   1 ml/l Spurenelemente
(3) Antibiotika-Medium (für höhere Pilze):
   20 g/l Malzextrakt
   10 g/l Glucose
   2 g/l Hefeextrakt
   0,5 g/l (NH₄)₂SO₄
   50 *µ*g/ml Tetracyclin

Alle Medien enthielten 5 mM N-Acetyl-PPT und wurden nach dem Überimpfen für 3-5 Tage bei 28°C inkubiert.

Die Anreicherungskulturen wurden anschließend auf Deacetylierung von N-Acetyl-PPT getestet. Dazu wurden die Zellen bei 10000 rpm abzentrifugiert und die Überstände im Aminosäureanalysator (Biotronic LC 5001) auf Bildung von PPT untersucht. Nur die Chitin-Medium-Kulturen erwiesen sich als Deactylasepositiv. Nach Ausplattierung auf Chitin-Agar wurden von diesen Kulturen ingesamt 40 Einzelkolonien isoliert, in Chitin-Flüssigmedium rekultiviert und erneut auf Deacetlyase -Aktivität getestet. Dabei wurden 6 positive Isolate gefunden, aus denen durch nochmaliges Ausstreichen auf Agar-Platten und Weiterkultivieren von Einzelkolonien die aktiven Reinkulturen gewonnen werden konnten. Der Stamm mit der höchsten Deacetylase-Aktivität wurde als Stenotrophomonas sp, identifiziert (DSM-Hinterlegungs-Nr. DSM 9734).

### Beispiel 2: Klonierung und Sequenzierung des N-Acetyl-PPT-Deactylase-Gens aus Stenotrophomonas sp.

Die für die Arbeiten benutzten molekularbiologischen Standardmethoden sind bei Maniatis et al. 1982, Molecular Cloning: a laboratory manual, Cold Spring Harbor Laboratory, Cold Spring Harbor, N.Y. beschrieben.

Genomische DNA aus Stenotrophomonas sp. wurde nach der Methode von Meade et al., 1982, J. Bacteriol. 149:114-122 präpariert. Nach partiellem Verdau mit Sau3A wurde die 5-10-kb Größenfraktion isoliert und in den mit BamHI geschnittenen lambda-ZAP-Express-Vektor von Stratagene (ZAP Express Vector Kit, Katalog Nr. 239201, Instruction Manual) ligiert. Mit dem verpackten Ligationsansatz wurde eine primäre lambda-Phagen-Bank hergestellt und anschließend amplifiziert. Daraus wurde mit Hilfe des pBK-CMV Phagemid-Systems von Stratagene (Katalog-Nr. 212209, Instruction Manual) eine Phagemid-Expressionsbank in Escherichia coli angelegt. Diese Genbank wurde durch Aktivitätsscreening mit [¹⁴C]-N-Acetyl-L-PPT als Substrat auf das

Vorhandensein des Deactylase-Gens geprüft. Zu diesem Zweck wurden 2000 Einzelklone in Miktrotiterplatten überimpft und über Nacht in LB-Medium bei 37°C mit 0,2 mM Isopropylthiogalaktosid (IPTG) als Induktor und 50 *µ*g/ml Kanamycin als Resistenzmarker angezogen. Die Zellen wurden abzentrifugiert und in je 10 *µ*l 1 mM [¹⁴C]-N-Acetyl-L-PPT über Nacht bei 28°C inkubiert.

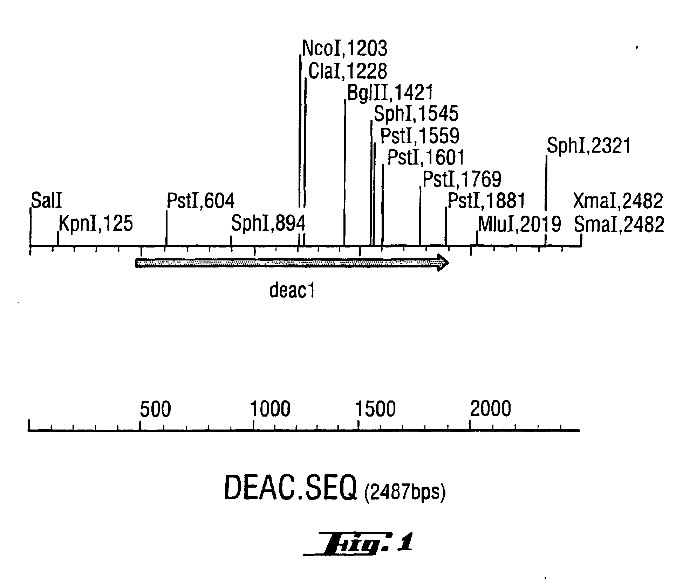

Anschließend wurden die Ansätze in Achterpools durch Dünnschichtchromatographie und Autoradiographie auf Umsetzung von N-Acetyl-PPT in PPT analysiert (siehe EP 531 716, Beispiel 8) und bei positivem Befund die Einzelklone nachgetestet. Mit diesem Verfahren konnte ein positiver Klon mit einem 6-kb Insert aus 1920 Transformanten selektiert werden. Dieses DNA-Fragment wurde durch Restriktionskartierung näher charakterisiert. Durch Subklonierung von Restriktionsfragmenten des Inserts in pUC18/19 und Transformation der rekombinanten Plasmide in E. coli konnte mit Hilfe des oben beschriebenen Aktivitätstests das deac-Strukturgen auf einem 2,5-kb (2487 Basenpaare) Sall/Smal-Fragment lokalisiert werden (Abb. 1). Die Aktivität war von der Orientierung des Fragmentes relativ zum lac-Promoter des Vektors abhängig.

Das 2,5-kb Fragment wurde mit der Sanger-Methode (Sanger et al. 1977, Proc. Natl. Acad. Sci USA 74:5463-5468) auf beiden Strängen sequenziert. Das gesamte Fragment hat eine Länge von 2487 Nukleotiden (siehe SEQ ID NO. 1). Der offene Leserahmen von 1494 Nukleotiden Länge beginnt mit Nukleotid-Nr.367 und endet mit Nr. 1860.

Expressionstudien mit PCR-Subfragmenten aus diesem Bereich in E. coli zeigten, daß das aktive Deacetylase-Protein von einem 1365 Nukleotide langen Bereich beginnend mit dem ATG-Startcodon in Position 496 und endend mit dem TGA-Stopcodon in Position 1860 codiert wird. Die aus der DNA-Sequenz abgeleitete Aminosäuresequenz stellt ein Polypeptid von 454 Aminosäuren (Seq ID NO 2) mit einem kalkuliertem Molekulargewicht von 48,1 kDa dar.

Eine Homologiesuche in den EMBL-, DNA- und Protein-Datenbanken ergab Ähnlichkeiten mit der in dieser Anmeldung zum ersten Mal beschriebenen N-Acetyl-PPT-Deacetylase aus Comamonas acidovorans (37,4 % identische Aminosäuren), der Hippurat-Hydrolase aus Campylobacter jejuni (33,9 %) und N-Acyl-L-Amidohydrolase aus Bacillus stearothermophilus (33,7 %). Im folgenden wird das aus Stenotrophomonas isolierte Gen als deac 1 bezeichnet. Das entsprechende Protein wird als Deac 1 bezeichnet. Aufgrund der Homologie kann man davon ausgehen, daß auch die genannten Hippurathydrolasen und Amidohydrolasen in Kombination mit gewebespezifischen Promotoren und einer anschließedend Behandlung vorteilhaft zur Herstellung von Pflanzen mit selektiv zerstörbaren Geweben, insbesondere männlich und/oder weiblich sterilen Pflanzen, genutzt werden können.

### Beispiel 3: Charakterisierung des Deac1-Proteins

Zur Überexpression und Reinigung der Deactylase aus Stenotrophamonas sp. (deac1) wurde das Glutathion-S-Transferase (GST)-Gen-Fusionsvektor-System von Pharmacia (Katalog Nr. 27-4581-01, Katalog-Nr. 27-4570-01) verwendet. Die Methode ist bei Smith and Johnson, 1988, Gene 67:31 beschrieben. Die Reinigung des Fusionsproteine erfolgt durch Affinitätschromatographie an Glutathion-Sepharose-4B.

Das funktionelle deac-Gen wurde als 1,4-kb BamHI/Sall-PCR-Fragment in den GST-Fusionsvektor pGEX-4T-2 unter Kontrolle des lac-Promoters umkloniert. Die Expression von rekombinantern Fusionsprotein wurde durch Zugabe von 0,1 mM IPTG induziert. Durch SDS/Polyacrylamid-Elektrophorese von Rohextrakten der E. coli-Transformanten konnte das Fusionsprotein als 74-kDa-Bande nachgewiesen werden.

Zur Proteinreinigung wurden die Zellen aus einer 2 l -Kultur einer induzierten, expressionspositiven E. coli Transformanten mit Ultraschall aufgeschlossen und der Extrakt anschließend durch Zugabe von 1 % Triton-X-100 solubilisiert. Der Überstand wurde an Glutathion-Sepharose-4B- gebunden. Nach mehrmaligem Waschen mit PBS-Puffer (140 mM NaCl, 3 mM KCL, 10 mM Na₂HPO₄, 2 mM KH₂PO₄, pH = 7,3) wurde das an die Sepharose-Matrix gebundene Fusionsprotein mit Thrombin gespalten. Das Eluat enthielt als einzige Proteinbande ein Spaltprodukt von 48-kDa (nach denaturierender SDS/Polyacrylamid-Elektrophorese), welches im Enzym-Assay (siehe unten) als N-Acetyl-PPT-Deacetylase identifiziert werden konnte. Mit der beschriebenen Methode ließen sich aus 21 Bakterienkultur 200 *µ*g Deacetylase-Protein zur Homogenität reinigen.

Die Aktivität des Deacetylase-Proteins wurde mit den zwei folgenden Assays gemessen:
(1) Radioaktiver Test: je 2,5 *µ*g gereinigtes Enzym wurde in 10 *µ*l Ansätzen in PBS-Puffer mit 0,1 mM [¹⁴C]-N-Acetyl-L-PPT für 15 min. bei 37°C inkubiert, Anschließend wurden die Proben 1:6 in 5 mM KH₂PO₄, 10 % Methanol, pH = 1,92 verdünnt und in der HPLC mit Radioaktivitätsdetektor analysiert (Trennsäule: Spherisorb SAX, Laufmittel: 5 mM. KH₂PO₄, 10 % Methanol, pH = 1,92, Flußrate: 0,5 ml/min). Unter diesen Bedingungen eluiert [¹⁴C]-L-PPT bei 4,5 min. und [¹⁴C]-N-Acetyl-L-PPT bei 6,5 min. Die spezifischen Deacetylase-Aktivitäten wurden in [nmol [¹⁴C]-L-PPT/min/mg Protein] ermittelt. Zur Ermittlung des pH-Optimums wurden die Ansätze in einem Puffer-System aus je 40 mM Bis-Tris, Tris und Caps zwischen pH = 6 und 9 inkubiert. Für die Kₘ-Wert-Messung wurden 3fach-Bestimmungen mit Konzentrationen von [¹⁴C]-N-Acetyl-L-PPT zwischen 0,01 mM und 1 mM bei pH = 8,0 in Anwesenheit von 1 mM CoCl₂ durchgeführt.
(2) Nicht-radioaktiver Test: Zur Untersuchung der Substratspezifität wurden je 5 *µ*g gereinigtes Enzym in 20 *µ*l Ansätzen in PBS-Puffer mit je 25 mM einer bestimmten N-Acetyl- bzw. N-Acyl-Aminsäure für 60 min. bei 28°C inkubiert. Anschließend wurden die Proben auf Bildung der freien Aminosäuren im Aminosäurenanalysator gemessen (Biotronic LC 5001). Die spezifischen Aktivitäten wurden in [nmol Aminosäure/min/mg Protein] ermittelt. Für die N-Acetyl-PPT-Deacetylase wurden ein pH-Optimum von pH = 8 (siehe Tab. 1) und ein Temperatur-Optimum von 37°C (siehe Tab. 2) ermittelt. Die kinetischen Messungen ergaben ein Kₘ-Wert von 670 *µ*M. Durch Zugabe von 1 mM CoCl₂ ließ sich die Enzymaktivität um ca. 20 % steigern.

**Tab. 1: pH-Optimum der N-Acetyl-PPT-Deacetylase**

| pH-Wert | Spez. Aktivität [mol/min/mg Protein] |
|---|---|
| 6 | 2,4 |
| 7 | 7,2 |
| 8 | 12,0 |
| 9 | 8,5 |

**Tab. 2: Temperatur-Optimum der N-Acetyl-PPT-Deacetylase**

| Temperatur [°C] | Spez. Aktivität [nmol/min/mg Protein]: |
|---|---|
| 28 | 9,6 |
| 37 | 16,8 |
| 50 | 14,9 |
| 60 | 4,3 |

Die Ergebnisse der Messungen der Substratspezifität sind in Tab. 3 dargestellt.

Das Enzym besitzt ein relativ breites Substratspektrum. Die höchsten Umsetzungen wurden mit Hippursäure (N-Benzoyl-Glycin) und N-Acetyl-L-Glutamat erzielt. Die Affinität für N-Acetyl-L-PPT liegt ungefähr 50 % unter der für die beiden oben genannten Substrate. Die Deacetylase besitzt eine ausschließliche Spezifität für N-Acetyl-L-Aminosäuren. Mit den entsprechenden D-Enantiomeren konnten keine Reaktionen beobachtet werden.

**Tab. 3: Substratspezifität der N-Acetyl-PPT-Deacetylase**

| Substrat¹ | Rel. Aktivität² [%] |
|---|---|
| Hippursäure (N-Benoyl-Glycin) | 100 |
| N-Ac-Phosphinothricin | 43 |
| N-Ac-Ornithin | 37 |
| N-Ac-Methionin | 0 |
| N-Ac-Tryptophan | 0,4 |
| N-Ac-Phenylalanin | 24 |
| N-Ac-Tyrosin | 11 |
| N-Ac-Glutaminsäure | 100 |
| N-Ac-Glutamin | 30 |
| N-Ac-Glycin | 59 |
| N-Ac-Histidin | 43 |
| N-Ac-Leucin | 33 |
| N-Ac-Valin | 2 |
| N-Ac-Serin | 4 |
| N-Ac-Prolin | 0 |

| | |
|---|---|
| ¹: Bei allen Verbindungen handelt es sich um die L-Enantiomere. ²: Die mit Hippursäure gemessene spezifische Aktivität wurde gleich 100% gesetzt und die anderen Werte darauf bezogen. | |

### Beispiel 4: Konstitutive Expression des deac1-Gens in Tabak

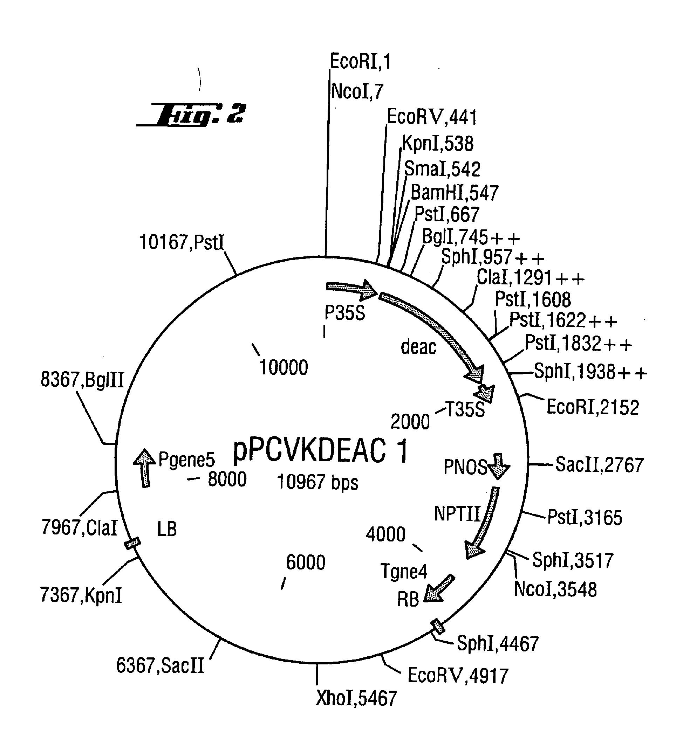

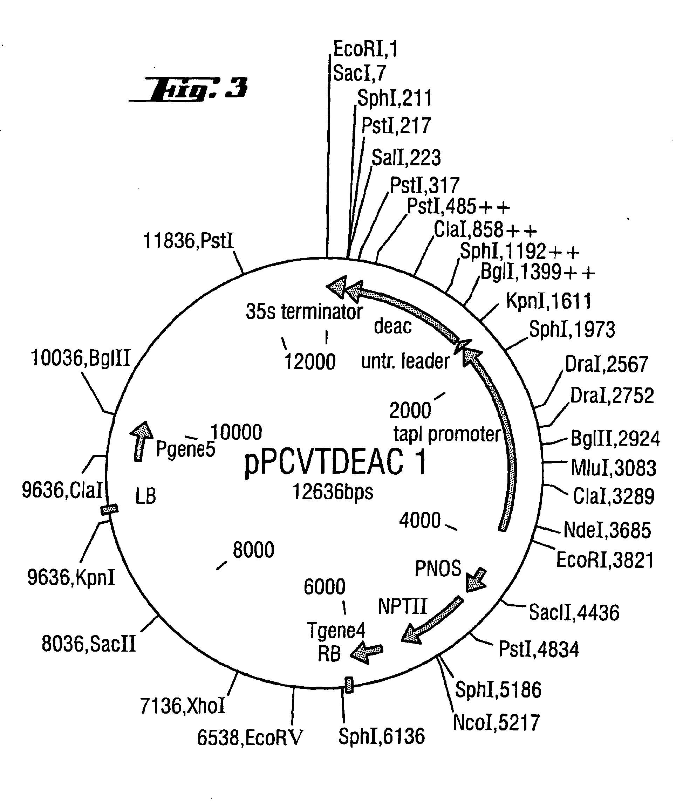

Das deac1-Strukturgen wurde als 1,4-kb BamHI/Sall-PCT-Fragment in den binären Vektor pPCV801 (Koncz and Schell, 1986, Mol. Gen. Genet. 204; 383-396) unter Kontrolle des 35S-Promoters umkloniert. Das entstandene Plasmid pPCVKDEAC1 (Abb. 2) mit dem Expressionsvektor 35S-Promoter-deac1-Strukturgen-35S-Terminator wurde unter Verwendung von Standard methoden in Agrobacterium tumefaciens (Stamm ATHV) transformiert. Blattstückchen von Tabak (Nicotiana tabacum) wurden mit den rekombinanten Agrobakterien nach der Methode von Horsch et al., 1985, Science 227: 1229-1231 transformiert und auf Kanamycin-Medium selektiert, 18 unabhängige Tabak-Transformanten wurden regeneriert und auf Expression der Deacetylase in den Blättern getestet. Im Falle einer Aktivität des Proteins in den transgenen Pflanzen war mit einer Empfindlichkeit der Blätter gegen N-Acetyl-PPT zu rechnen, weil die Substanz in der Pflanze aufgrund der enzymatischen Aktivität der Deacetylase dann in den herbiziden Wirkstoff Phosphinothricin umgewandelt wird.

In einem Tropfenversuch wurden die Blätter der transgenen Pflanzen sowie von mehreren nicht-transgenen Kontrollpflanzen mit je 5 *µ*l der folgenden Konzentrationen von N-Acetyl-D,L-PPT behandelt: 4 mg/ml (= 15 mM), 1 mg/ml (= 3,75 mM), 0,4 mg/ml (= 1,5 mM), 0,1 mg/ml (= 4,38 mM). Die Behandlungsstellen wurden nach 1-2 Wochen auf Aufhellungen bzw. Nekrosenbildung untersucht. Gleichzeitg wurde die N-Acetyl-PPT-spezifische Deacetylase-Aktivität der Transformanten, sowie der Kontrollpflanzen in Rohextrakten gemessen. Dazu wurden je 100 mg Blattmaterial in 200 *µ*l PBS-Puffer homogenisiert, die Zelltrümmer abzentrifugiert und die proteinhaltigen Überstände über Nacht bei 4°C gegen den gleichen Puffer dialysiert. Je 70 *µ*l dieser Proben wurden mit 0,1 mM [¹⁴C]-N-Acetyl-L-PPT über Nacht bei 37°C inkubiert. Die Ansätze wurden anschließend in der HPLC, wie oben beschrieben, auf Bildung von [¹⁴C]-L-PPT analysiert.

Die Testergebnisse der Tropfenversuche und der Aktivitätstests sind für ausgewählte Pflanzen in Tab. 4 dargestellt. Es zeigt sich, daß ca. 60 % der Transformanten das funktionelle Deacetylase-Protein exprimieren und den entsprechenden Phänotyp ausprägen.

**Tab. 4: Empfindlichkeit von pPCVKDEAC1-Pflanzen gegen N-Acetyl-PPT und Deacetylase-Aktivität in Rohextrakten**

| Pflanze | Tropfenversuch mit: | | Deac.-Aktivität im Rohextrakt | | | |
|---|---|---|---|---|---|---|
| | N-Ac-D,L-PPT 4 mg/ml | | 1 mg/ml | 0,4 mg/ml | 0,1 mg/ml | [% L-PPT]¹ |
| | Beobachtungen (Blatt:) | | | | | |
| Kontrolle | | - | - | - | - | 0 |
| pPCVK9 | | + + + | + | - | - | 14,2 |
| pFCVK14 | verkrümmt | + | + | - | - | 12,1 |
| pPCVK15 | verkrümmt | + + | + | + | - | 36,0 |
| pPCVK16 | verkrümmt | + | + | - | - | 4,7 |
| pPCVK17 | verkrümmt | + | + | - | - | 4,3 |

| | | | | | | |
|---|---|---|---|---|---|---|
| ¹: bezogen auf die eingesetzte Radioaktivität ([¹⁴C]-N-Acetyl-L-PPT) + + +: starke Nekrose + + : deutliche Schädigung + : schwache Aufhellung - : keine Symptome | | | | | | |

### Beispiel 5: Tapetum-spezifische Expression des deac1-Gens in Tabak

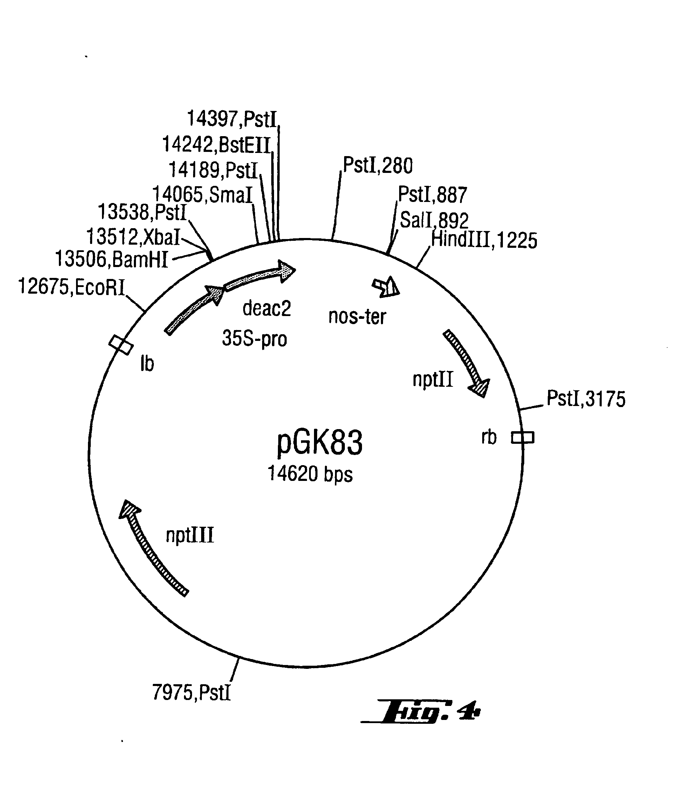

Unter Verwendung von Standardmethoden wurde der Tapetum-spezifische Promoter des tap1-Gens aus Löwenmäulchen (Antirrhinum majus) als 2,2-kb EcoRI/BamHI-Fragment mit dem deac1-Strukturgen (1,4-kb BamHI/SalI-PCR-Fragment) und dem 35S-Terminator im Vektor pUC18 fusioniert. Die so entstandenen Expressionskassette tap1-Promoter-deac-Strukturgen-35S-Terminator wurde als 3,8-kb EcoRI-Fragment an Stelle der 35S-Promoter/Terminator-Region in den binären Vektor pPCV801 einkloniert (Plasmid pPCVTDEAC1, Abb. 3). Die Transformation von Tabak wurde wie in Beispiel 4 beschrieben durchgeführt.

Im Falle einer Tapetum-spezifischen Expression der Deacetylase sollten durch Behandlung der Blütenknospen mit N-Acetyl-PPT männlich sterile Blüten induziert werden können, Die Umwandlung des PPT-Derivates in den herbiziden Wirkstoff Phosphinothricin führt zu einer selektiven Schädigung des Tapetum-Gewebes, wodurch die Entwicklung funktioneller Pollen verhindert wird.

Jeweils nur eine Seite der Infloreszenzen (der gesamte Bereich der sich entwickelnden Blütenknospen) wurde mit N-Acetyl-PPT behandelt, die andere Seite der Influoreszen blieb unbehandelt, um sicherzustellen, daß die zu beobachtenden Phänomene nicht auf Mißbildungen der jeweiligen Pflanzen zurückzuführen sind. Die Behandlung wurde zu einem Zeitpunkt durchgeführt, zu dem die einzelnen Knospen nicht größer als 5 mm waren(aktive Phase für Tapetum-Promoter). Die Tabak-Transformanten sowie mehreren NT Sam NN-Kontrollpflanzen wurden innerhalb einer Woche 3x mit 0,2 % N-Acetyl-D,L-PPT (= 7,5 mM) / 0,1 % Genapol (Netzmittel) behandelt. Nach dem Aufblühen der Knospen (ca, 9-11 Tage nach Abschluß der Behandlung) wurden die Pflanzen auf das Auftreten männlich steriler Blüten untersucht.

Gleichzeitig wurde die N-Acetyl-PPT-spezifische Deacetylase-Aktivität in unreifen Antheren der Transformanten, sowie der Kontrollpflanzen gemessen. Dazu wurden die unreifen Antheren aus ca. 5 mm großen Blütenknospen präpariert und über Nacht bei Raumtemperatur in je 50 *µ*M [¹⁴C]-N-Acetyl-L-PPT-Lösung inkubiert. Anschließend wurden die Antheren 1x in 500 *µ*l PBS-Puffer gewaschen und dann in 50 *µ*l PBS-Puffer homogenisiert. Nach Abzentrifugieren der Zelltrümmer wurden die Überstände in der Speed-Vac eingeengt und die Pellets in je 30 *µ*l HPLC-Puffer (5 mM KH₂PO₄, 10 % Methanol, pH = 1,921 aufgenommen. Die Ansätze wurden in der HPLC, wie oben beschrieben auf Bildung von [¹⁴C]-L-PPT analysiert.

Die Ergebnisse der Blütenbehandlung und der Aktivitätstests sind für ausgewählte Pflanzen in Tab. 5 dargestellt. In fast allen Transformanten konnte eine Antheren-spezifische Deacetylase-Aktivität festgestellt werden. Bei 3 Transformanten wurden auf der mit N-Acetyl-PPT-behandelten Seite der Blütentraube männlich sterile, d. h. es wurde kein Pollen gebildet, bzw. stark pollenreduzierte Blüten beobachtet. Die Wirkung auf neue, reifende Knospen hielt über einen Zeitraum von ca, 3 Wochen an. Die unbehandelten Blüten der Transformanten, sowie die behandelten Blüten der Kontrollpflanzen waren in allen Fällen fertil. Bei den männlich sterilen Blüten war keine Samenbildung feststellbar. Eine gezielte Fremdbestäubung der männlich sterilen Blüten war jedoch möglich, wodurch bewiesen wurde, daß die weiblichen Blütenteile ihre volle Funktionsfähigkeit behalten haben (Nacken et al, 1991, Mol.Gen. Genet. 229: 129-136).

**Tab. 5: Induzierte männliche Sterilität bei pPCVTDEAC1-Pflanzen durch Blütenbehandlung mit N-Acetyl-PPT und Deacetylase-Aktivität in Antheren**

| Pflanze | Anzahl von sterilen bzw. | Deac.Aktivität in Antheren | |
|---|---|---|---|
| | pollenreduzierten Blüten | [% L-PPT]¹ | |
| | behandelt | unbehandelt | |
| Kontrolle | 0 | 0 | 0 |
| pPCVT14 | 18 | 0 | 70,0 |
| pPCVT15 | 0 | 0 | 13,8 |
| pPCT16 | 10 | 0 | 20,4 |

| | | | |
|---|---|---|---|
| ¹: bezogen auf die eingesetzte Radioaktivität ([¹⁴C]-N-Acetyl-L-PPT) | | | |

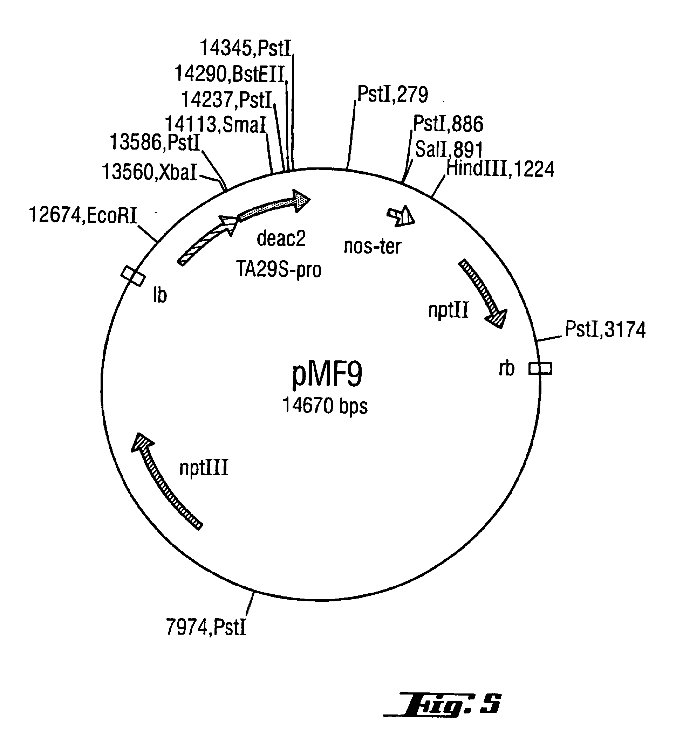

- Abb. 1: Restriktionskarte des 2,5-kb Sall/SamHI-Fragmentes, welches die N-Acetyl-PPT-spezifische Deacetylase-Aktivität vermittelt. Position und Orientierung des deac1-Strukturgens sind durch Pfeil markiert (BP = Basenpaare).
- Abb. 2: Karte des Plasmids pPVCKDEAC1 zur konstitutiven Expression des deac-Gens in Pflanzen
- Abb. 3: Karte des Plasmids pPCVTDEAC1 zur Tapetum-spezifischen Expression des deac-Gens in Pflanzen
- Abb, 4: Karte des Plasmids pGK83
- Abb. 5: Karte des Plasmids pMF9

## Patentansprüche

1. DNA-Molekül kodierend für ein Protein mit hoher N-Acetyl-Phosphinothricin-Deacetylase-Aktivität, isolierbar aus *Stenotrophomonas sp.* hinterlegt unter DSM 9734.

2. Protein, das von einem DNA-Molekül gemäß Anspruch 1 kodiert wird.

3. Mikroorganismus *Stenotrophomonas sp.,* hinterlegt unter DSM 9734.

4. Pflanzenzelle, die ein DNA-Molekül gemäß Anspruch 1 enthält.

5. Pflanze, die ein DNA-Molekül gemäß Anspruch 1 enthält.

6. Verfahren zur Herstellung transgener Pflanzen mit gezielt zerstörbaren Teilen, umfassend:
a) die Transformation einer Pflanzenzelle mit einem DNA-Molekül gemäß Anspruch 1, welches sich unter der Kontrolle eines gewebe-spezifischen Promotors befindet; und
b) die Regeneration von Pflanzen aus den nach (a) erhaltenen Pflanzenzellen, wobei die erhaltenen Pflanzen Pflanzenteile besitzen, die selektiv zerstört werden können.

7. Verfahren gemäß Anspruch 6 zur Herstellung männlich steriler Pflanzen, **dadurch gekennzeichnet, dass**
a) der gewebe-spezifische Promotor ein Tapetum-spezifischer Promotor ist; und
b) die Behandlung mit N-Acetyl-Phosphinothricin das Tapetum-Gewebe schädigt.

## Claims

1. A DNA molecule coding for a protein having high N-acetyl-phosphinothricin deacetylase activity, isolable from *Stenotrophomonas sp.* deposited under DSM 9734.

2. A protein coded for by a DNA molecule according to claim 1.

3. A microorganism *Stenotrophomonas sp.,* deposited under DSM 9734.

4. A plant cell containing a DNA molecule according to claim 1.

5. A plant containing a DNA molecule according to claim 1.

6. A process for preparing transgenic plants having parts which can be destroyed specifically, comprising the steps of:
a) transforming a plant cell with a DNA molecule according to claim 1 which is under the control of a tissue-specific promoter; and
b) regenerating plants from the plant cells obtained in (a), the plants obtained having parts which can be destroyed selectively.

7. A process according to claim 6 for producing male sterile plants, **characterized in that**
a) the tissue-specific promoter is a tapetum-specific promoter; and
b) treating with N-acetyl-phosphinothricin damages the tapetum tissue.

## Revendications

1. Molécule d'ADN codant pour une protéine ayant une activité élevée de la N-acétylphosphinothricine-déacétylase, que l'on peut isoler à partir de *Stenotrophomonas sp.* déposé sous DSM 9734.

2. Protéine, qui est codée par une molécule d'ADN selon la revendication 1.

3. Microorganisme *Stenotrophomonas sp.* déposé sous DSM 9734.

4. Cellule de plante qui contient une molécule d'ADN selon la revendication 1.

5. Plante qui contient une molécule d'ADN selon la revendication 1.

6. Procédé pour la préparation de plantes transgéniques avec des parties destructibles de manière ciblée, comprenant :
a) la transformation d'une cellule de plante avec une molécule d'ADN selon la revendication 1, qui se trouve sous le contrôle d'un promoteur spécifique de tissu; et
b) la régénération de plantes à partir de cellules de plantes obtenues selon (a), les plantes obtenues possédant des parties de plantes qui peuvent être détruites sélectivement.

7. Procédé selon la revendication 6, pour la préparation de plantes à stérilité mâle, **caractérisé en ce que**
a) le promoteur spécifique de tissu est un promoteur spécifique de tapetum ; et
b) le traitement par la N-acétylphosphinothricine qui endommage le tissu de tapetum.
